# EUROPEAN PATENT SPECIFICATION

(11) **EP 0 514 451 B1**
(45) Date of publication and mention of the grant of the patent: **15.01.1997**
(21) Application number: 91904156.6
(22) Date of filing: 04.02.1991
(51) Int. Cl.: A23L 1/302, A23L 1/304, A61K 38/43, A61K 33/00

(54) **DIETARY SUPPLEMENTS COMPRISING VITAMINS AND MINERALS**
VITAMINE UND MINERALIEN ENTHALTENDE NAHRUNGSZUSÄTZE
COMPLEMENTS ALIMENTAIRES COMPRENANT DES VITAMINES ET DES SELS MINERAUX

(30) Priority: 05.02.1990 US 475641
(43) Date of publication of application: 25.11.1992
(73) Proprietor: LifeScience Corporation, Austin, Texas 78730 (US)
(72) Inventor: SLAGA, Thomas, J., Austin, TX 78746 (US); DELUCA, Daryl, L., Sugarland, TX 77478 (US); SPARKS, William, S., Bellaire, TX 77401 (US)
(74) Representative: Grünecker, Kinkeldey, Stockmair & Schwanhäusser Anwaltssozietät
(86) International application number: US9100719
(87) International publication number: WO9111117

(56) References cited:
- EP-A- 0 129 032
- EP-A- 0 259 167
- FR-A- 2 244 468
- FR-A- 2 569 536
- FR-A- 2 612 373

## Description

The present invention relates to a daily dietary multi-vitamin and mineral supplement for a human and an oral sustained release tablet including said supplement.

Vitamins and minerals may be broadly defined as substances that are essential for the maintenance of normal metabolic function but are not synthesized in the body. These substances must be furnished from an exogenous source. A healthy individual ingesting a well-balanced diet receives adequate amounts of vitamins and minerals from food. Vitamins obtained in this manner are not generally regarded as drugs.

However, there are many situations in which the concentration of one or more vitamins or minerals in the body tissues may be suboptimal. When such circumstances arise or can be anticipated, it is the common practice to administer vitamins or minerals in a chemically pure form. When employed in this manner, vitamins and minerals may be regarded as drugs and a knowledge of their pharmacological properties is desirable. For example, large doses of various vitamins are frequently employed for the treatment of disorders that are not etiologically related to vitamin deficiency. The implication is that such large doses may exert pharmacodynamic actions that are useful in therapy.

It should be noted, however, that excessive administration of certain vitamins, notably vitamin A, can cause untoward toxic effects.

In today's fast paced society many physicians and dieticians believe that a daily dietary vitamin and mineral supplement acts prophylactically to prevent pathologies associated with vitamin and mineral deficiencies. These supplements typically include several vitamins and minerals, and are referred to as multi-vitamin formulations. Examples of these preparations include One-A-Day® and Centrum® multi-vitamins. Researchers have suggested that multi-vitamins which include large quantities of vitamins and minerals are effective both for prophylactic purposes and for the treatment of an enormous variety of illnesses. Many millions of individuals living in the United States regularly ingest multi-vitamin preparations.

Some of these multi-vitamin preparations contain amounts of vitamins and minerals that are in excess of the RDA daily requirements. In the case of the water-soluble vitamins, there is little harm done to the body because of the low toxicity of this class of compounds. The low toxicity of the water-soluble vitamins is attributable to the fact that excess quantities of these substances are rapidly excreted in the urine. In the case of the lipid-soluble vitamins, the compounds accumulate in the body fat and can cause untoward toxic effects.

It is extremely important to distinguish between supplemental and therapeutic vitamin preparations. The latter contains much larger quantities of the individual vitamins and minerals than do the supplemental preparations. The present invention is directed to supplemental preparations.

The use of dietary multi-vitamin supplements should be considered by the physician in a wide variety of situations. Such situations may result from (1) inadequate vitamin intake, (2) mal-absorption syndromes, and (3) increased tissue requirements.

Vitamin deficiency due to an inadequate intake arises in a variety of circumstances. There are still large parts of the world and even considerable areas in the United States where, owing to poverty, the population eats a diet inadequate in terms of vitamin content. Moreover, there are areas where the prevalence in the diet of one particular type of food results in a relatively high incidence of vitamin deficiencies. Eccentric diets resulting from psychiatric disturbance, individual idiosyncrasies, religious beliefs, or food fads are other major causes of vitamin deficiency. A decrease in food consumption because of excess use of alcoholic beverages, poor appetite, dieting to combat obesity, or restricted diets prescribed by physicians for the management of specific diseases represents a further group of situations in which vitamin deficiency can arise.

A disturbance in absorption of vitamins is also seen in a variety of conditions. Examples are diseases of the liver and biliary tract, prolonged diarrhea from any cause, hyperthyroidism, pernicious anemia, and a variety of other disorders of the digestive system. Moreover, since a substantial proportion of certain vitamins is provided by the bacteria of the gastrointestinal tract, treatment with antimicrobials that alter the intestinal bacterial flora may lead inevitably to a decreased vitamin absorption.

Increased tissue requirements for vitamins also occur under a variety of conditions so that a nutritional deficiency may develop on a diet that had previously been adequate. Such situations can occur in both health and disease. In healthy individuals, for example, there is a greater requirement for various nutrients, including vitamins, during growth, during periods of hard physical work, exercise, during pregnancy, lactation, and menstruation. Diseases associated with an increased metabolism, such as hyperthyroidism, and conditions accompanied by fever or tissue wasting also increase the body's requirements for vitamins. There is also good evidence that vitamin requirements increase during stress and after injury.

Multiple-vitamin therapy, using supplemental dietary multi-vitamins, is desirable in a variety of situations. Since deficiency of a single vitamin is rarely encountered clinically, it is customary practice in cases of suspected vitamin deficiency, as well as in prophylactic treatment, to recommend that multiple-vitamin therapy be administered. Typical of the multi-vitamins available are One-A-Day and Centrum multi-vitamins. (Table 1). Multi-vitamins such as these provide a great number of the essential vitamins and minerals needed by healthy individuals. Further, these preparations include electrolytes, and trace elements. Recently, it has been determined that antioxidants, and free radical scavenging agents are protective against the development of certain cancers. Although, multi-vitamin preparations currently available include many needed vitamins and minerals, they do not include the antioxidants and/or free radical scavengers which have been shown to enhance the immune system and to prevent certain cancers, cardiovascular disorders, inflammatory diseases and to slow ageing. Further, currently available multi-vitamin preparations do not include cardio-protective inositol or Omega-3 fish oils. Still further, several enzymes, amino acids, and dietary supplements which are known to be necessary and beneficial in supplementing an individual's diet are not included in presently available multi-vitamins. Accordingly, a new multi-vitamin dietary supplement is needed which incorporates many of these beneficial ingredients into a stable pharmaceutical preparation.

FR-A-1 298 301 discloses a nutritive product containing fat-soluble vitamins wherein the vitamins are very stable and highly digestible. This reference proposes to use dry carriers in order to prevent the nutritive products containing the fat-soluble vitamins from deterioration by oxidation. In particular, the conservation of vitamin A for prolonged periods, even at elevated temperatures is contemplated.

This object is realized by providing products having a homogeneous solid phase containing a vitamin, an antioxidant and ethylcellulose resin. The vitamin is selected from vitamin A, precursors of vitamin A such as carotene and certain forms of vitamin E. The antioxidant can be selected from propyl gallate, BHA and others. The examples of this reference show that the nutritive products according to this invention contain either vitamin A and derivatives thereof respectively or vitamin E, and antioxidant and ethylcellulose.

FR-A-2 244 468 discloses a food product and food additive exhibiting a protective activity against cancer which consist of an antioxidant selected from the group consisting of vitamin C, vitamin E and natural or synthetic antioxidants and an organic body containing sulfur such as an amino acid, a peptide or a sulfur containing protein.

The present invention concerns development of a multi-vitamin dietary supplement formulation which includes several dietary supplements which are cardio- and cancer-protective as well as immunostimulatory. By including these cardio- and cancer-protective immunostimulatory compounds into a multi-vitamin formulation, the present invention advantageously provides the general population the benefits of recent scientific research which has discovered that several agents are, for example, cardio- and cancer-protective. Accordingly, it is believed that the inventive formulations will provide the general population with an improved multi-vitamin formulation capable of reducing the incidence of diseases such as heart disease and cancer while enhancing longevity and the immune system.

The present invention is directed to a formulation including vitamins, minerals and other beneficial supplements. More specifically, the invention is directed to a formulation containing vitamins and minerals in association with antioxidants, fish oils, food concentrate containing naturally occurring anti-oxidizing enzymes such as catalase and superoxide dismutase, for example, and amino acids. The formulations of the present invention exploit synergistic relationships which have not previously been reported.

One aspect of the present invention is directed to a multi-vitamin and mineral supplement which includes at least one antioxidant selected from the group consisting of BHT, BHA, propyl gallate, and sodium benzoate, vitamin A, vitamin E and beta carotene.

Several of the antioxidants, vitamins and minerals contained in the preferred formulation have been shown to inhibit the induction of cancer at several stages in the multistage process. For example, B-carotene, glutathione, cysteine, BHA, BHT, Vitamins A, C and E, as well as selenium, inhibit the tumor initiation, promotion and progression stages of cancer. The synergistic effects of BHA and Vitamin E, for example, are related to the observations that BHA inhibits tumor promoter-induced polyamine synthesis, and Vitamin E inhibits promoter-induced hyperplasia, thus leading to a synergistic effect. Furthermore, Vitamins A, C and E, selenium, BHA, BHT, and propyl gallate have been shown to inhibit tumor initiation by counteracting the covalent binding of carcinogen to DNA and the mutagenic effect of carcinogens.

A further aspect of the present invention is directed to a multi-vitamin and mineral supplement including at least one antioxidant selected from BHT, BHA, propyl gallate and sodium benzoate, vitamin A, vitamin E, beta carotene, vitamin C, selenium, copper, zinc, manganese, omega-3 fish oil, inositol, PABA, bioflavonoids, reduced L-glutathione, L-cysteine, potassium sorbate or sorbic acid, sodium benzoate, taurine, D, L-methionine, L-glutamine, SOD, and catalase.

Preferably, the supplement of the present invention further includes (but is not limited to) the following vitamins and minerals in an amount equal to or greater than the minimum daily requirement in man: vitamin C, folic acid, vitamin B₁, vitamin B₂, niacinamide, vitamin B₆, vitamin B₁₂, vitamin D₃, biotin, pantothenic acid, vitamin K₁, calcium, iodine, potassium, iron, magnesium, chromium, molybdenum, vanadium, silicon, and boron.

A still further aspect of the present invention is directed to the production of a sustained-release tableted dietary supplement which is intended to be administered one to three times daily.

FIG. 1 graphically represents, as a function of % potassium released versus time, the dissolution of Centrum tablets in gastric juice.

FIG. 2 graphically represents, as a function of % potassium released versus time, the dissolution of the sustained release tablets of the present invention in intestinal juice.

FIG. 3 graphically represents, as a function of % ascorbic acid released versus time, the dissolution of the sustained release tablets of the present invention in intestinal juice.

FIG. 4 graphically represents, as a function of % niacinamide released versus time, the dissolution of the sustained release tablets of the present invention in intestinal juice.

FIG. 5 graphically represents, as a function of pyridoxine % released versus time, the dissolution of the sustained release tablets of the present invention in intestinal juice.

The present invention concerns a formulation of vitamins, minerals and other beneficial supplements. It is believed that the formulations of the present invention will not only extend longevity but also protect against certain cancers, immunological disorders and cardiovascular disorders in humans. Generally, the invention is directed to a formulation containing vitamins and minerals in association with antioxidants, fish oils, anti-oxidizing enzymes preferably contained in food concentrates, and amino acids. It is believed that the formulations of the present invention will be beneficial in treating or preventing several pathologies, including various vitamin deficiencies, certain cancers, immunological disorders, and cardiovascular disease.

The formulations of the present invention exploit some synergistic relationships which have not previously been reported. The formulations of the present invention are particularly well adapted as a daily dietary supplement.

Table 1 shows the anti-cancer effects of various components of the present formulation, individually and in synergistic combination.

**Table 1**

| **Effects of antioxidants on Skin Tumor Promotion**^{**a**} | |
|---|---|
| Antioxidant (mg) | Tumor Response (% Inhibition) |
| Control (DMBA + TPA) | 0 |
| BHA (0.5) | 35 |
| BHT (0.5) | 32 |
| Propyl gallate (0.5) | 40 |
| Vitamin E (0.1) | 25 |
| Vitamin C (0.5) | 30 |
| B-carotene (0.5) | 20 |
| Selenium (0.004) | 23 |
| BHA (0.5) + BHT (0.5) | 92 |
| BHA (0.5) + Vit E (0.1) | 86 |
| BHA (0.5) + Vit C (0.5) | 98 |
| Vit E (0.1) + Selenium (0.004) | 94 |
| Vit E (0.1) + B-carotene (0.5) | 94 |
| BHA (0.5) + B-carotene (0.5) | 87 |
| BHA(0.1)+BHT(0.1)+Vit E(0.1)+B-carotene(0.1) | 97 |

| | |
|---|---|
| ^{a}30 female mice (SENCAR) per group were used. The mice were initiated with 10 ηmoles of 7,12-dimethylbenzanthracene and promoted with 1 µg of TPA 2x/wk. The antioxidants were applied simultaneously with TPA for the duration of experiment (30 weeks). | |

Table 2 illustrates the effects of certain antioxidant components of the present formulation on all the known spontaneous tumor formations and longevity in mice.

**Table 2**

| **Effects of Antioxidants on Aging and Spontaneous Tumor Formation in Mice**^{**a**} | | |
|---|---|---|
| Antioxidants | Spontaneous Tumor Formation (% of Control) | Increase in Lifespan (%) |
| Normal Diet | 100 | - |
| BHA | 46 | 132 |
| BHT | 56 | 124 |
| Propyl gallate | 62 | 127 |

| | | |
|---|---|---|
| ^{a}100 mice (SENCAR) (50 males and 50 females) were used for each group. The groups receiving the antioxidants had them introduced in the normal laboratory chow (Wayne Research Animal Diet) at 0.5%. | | |

Core ingredients in the most preferred formulation of the present invention are antioxidants and/or free radical scavenging agents of different types which protect against many kinds of free radicals and unstable molecules. In some cases, they work synergistically with other antioxidants, vitamins, and minerals. The following are the Core ingredients:
bioflavonoids
L-glutathione (reduced)
L-Cysteine
Potassium sorbate/sorbic acid
BHA
BHT
Propyl gallate
Sodium benzoate
Taurine
D L-Methionine
L-glutamine
SOD and Catalase
   (preferably contained in food concentrate)

The significance and content of the Core ingredients may be described as follows:

Bioflavonoids are biologically active compounds that have been shown to act as antioxidants, protecting vitamin C and other components from oxidation. Certain bioflavonoids have been shown to have a protective effect against some cancers. The dosage of the bioflavonoid included is from about 2 to 200 mg, more preferably, from about 25 to about 150 mg, and most preferably, about 50 mg. Preferable sources of bioflavonoid include citrus bioflavonoid, vitamin P, vitamin P complex, rutin, orange peel bioflavonoid, grapefruit peel bioflavonoid, lemon bioflavonoid, lime bioflavonoid, flavonoids, kaempferol, narigenin, apigenin, naringin, naringenis, myricetin, delphinidin, quercetin, phloretin, cyanic, catechin, morin, phloridzin, phloretin, 3-hydroxyflavones, 3-deoxyflavonols, isorhamnetin, luteolin, tricin, chrysoeriol, hesperitin, eriodictyon, techtrochrysin, silybin, taxifolin, pinocembrim, galangin, robinin, diosmetin, kaempferide, fisetin, rhamnetin, and 3-0-methyl catechin. The most preferable source is, however, citrus bioflavonoid.

It is believed that glutathione may protect normal cells against radiation, free radicals and other toxic materials, and certain cancers. Functionally, glutathione is the proton donor used by glutathione peroxidase to remove free radicals. Forms of glutathione useful in the practice of the present invention include: L-glutathione, S-ethylglutathione, S-butylglutathione, S-decylglutathione, S-heptylglutathione, S-methylglutathione, S-propylglutathione, S-pentylglutathione, S-octylglutathione and S-nonylglutathione. However, most preferably, glutathione is included in the formulations of the present invention as reduced L-glutathione. The dosage of glutathione included to the inventive formulation is preferably from about 1 to about 20 mg, and most preferably, about 10 mg.

L-Cysteine is an amino acid which has antioxidant properties and plays an important role in glutathione peroxidase activity. Cysteine also appears to have certain anticancer activity. Preferably, cysteine is included in the present invention as L-cysteine HCl, L-cysteine, L-acetyl cysteine, L-cysteine ethylester monohydrochloride, DL-cysteine, DL-cysteine HCl, L-cysteine methylester. However, it is most preferred, that cysteine is included as L-cysteine HCl, in a dosage of from about 5 to about 100 mg, and more preferably, about 50 mg.

Potassium sorbate and/or Sorbic acid are useful as a mold and yeast prohibitor. Sorbic acid has been shown to increase the effectiveness of antioxidant mixtures by acting synergistically with them. The sorbic acid dosage is preferably from about 10 to 500 mg as potassium sorbate, sorbic acid, or sodium sorbate. Most preferably, however, the dosage of sorbic acid is about 200 mg as potassium sorbate.

In one important aspect of the present invention, the formulation includes at least one antioxidant. The antioxidant is selected from the group consisting of butylated hydroxyanisole, butylated hydroxytoluene, propyl gallate, and sodium benzoate. The inventive formulation may include one, or, more preferably, several of the preferred antioxidants in combination. According to one embodiment, the inventive formulation includes butylated hydroxyanisole (BHA), butylated hydroxytoluene (BHT), sodium benzoate, and propyl gallate. Sodium benzoate, BHA, BHT and propyl gallate are all antioxidants used to stabilize cosmetic, pharmaceutical, and food products. It has been reported that these agents protect normal cells against radiation, free radicals, toxic chemicals and chemical carcinogens. It has also been reported that these agents are protective against the induction of cancer by many cancer-causing agents in almost every tissue of the body. Further, the preferred antioxidants of the present invention have been reported to protect against cardiovascular disorders, enhance immune function and increase longevity. The antioxidants of the present invention work synergistically with several vitamins, minerals, amino acids, enzymes, and other antioxidants or free radial scavengers included in the formulation of the present invention as will be described in detail below.

According to one embodiment, BHA is included in a daily dietary multi-vitamin and mineral formulation for humans administered on a 1-3 times daily basis. Preferably, from 10 to 300 mg of BHA is included in combination with a number of vitamins and minerals. More preferably, however, the formulation includes from about 10 to about 150 mg of BHA, and most preferably, about 50 mg of BHA. BHA may be included in the inventive formulation as butylated hydroxyanisole, 3-tert-butyl-4-hydroxyanisole, or 2-tert-butyl-4-hydroxyanisole, but most preferably is butylated hydroxyanisole.

According to another preferred embodiment, BHT is included in a daily dietary multi-vitamin and mineral formulation. Preferably, from 10 to 300 mg of BHT is included in combination with a number of vitamins and minerals. More preferably, however, the formulation includes from about 10 to about 150 mg of BHT, and most preferably, about 50 mg of BHT.

According to further embodiment, propyl gallate is included in the daily dietary multi-vitamin and mineral formulation. Preferably, from 10 to 300 mg of propyl gallate is included in combination with a number of vitamins and minerals. More preferably, however, the formulation includes from about 10 to about 150 mg of propyl gallate, and most preferably, about 50 mg of propyl gallate.

Sodium benzoate, an antioxidant used to stabilize food and pharmaceutical products, according to another embodiment, is included in a daily dietary multi-vitamin and mineral formulation. Preferably, from 1 to 300 mg of sodium benzoate is included in combination with a number of vitamins and minerals. More preferably, however, the formulation includes from about 1 to about 150 mg of sodium benzoate, and most preferably, about 4 mg of sodium benzoate. As described above, one embodiment of the inventive formulation includes sodium benzoate. However, it has been determined that potassium benzoate, benzoic acid, calcium benzoate, cuprous benzoate, cupric benzoate, manganese benzoate, nickel benzoate, magnesium benzoate are all also useful in the practice of the present invention.

Taurine is an amino acid used by the liver for conjugation with bile acids. It has also been suggested that taurine strengthens and stabilizes cellular membranes. High concentrations of taurine are found in the brain. Taurine, it is believed, acts in concert with the preferred antioxidants of the present invention to provide increased protection against cancer and certain cardiovascular diseases. Preferably, taurine is included in the formulations of the present invention in a dosage of from 1 to 20 mg, and most preferably, about 10 mg.

Methionine is an essential amino acid which has antioxidant properties. Methionine is the principal source of methyl groups which are, in turn, transferred to many diverse acceptors in important physiological reactions, such as, methyl transferases. Methionine is preferably included in the formulations of the present invention as DL-methionine, L-methionine, S-adenosylmethionine, N-acetyl-methionine, S-methyl-DL-methionine-sulfonium chloride. However, more preferably, methionine is included as DL-methionine, in a dosage of from 1 to 20 mg, and most preferably, about 10 mg.

Glutamine is an amino acid which serves as an amino donor in many reactions. Glutamine is critical for the de novo synthesis of purine and pyrimidine nucleotides. These nucleotides are critical to normal cell function and replication. Preferably, glutamine is included in the formulations of the present invention as L-glutamine, DL-glutamine, or N-acetyl-L-glutamine. However, most preferably, glutamine is included as L-glutamine, in a dosage of from 1 to 20 mg, and most preferably, about 10 mg.

Superoxide dismutase (SOD) stabilizes the formulations of the present invention and is an antioxidant. SOD prevents the formation of singles oxygen, hydroxyl radicals and other reactive oxygen species. It is believed that SOD can protect against cancer, cardiovascular disorders and increase longevity. The dosage included of SOD (superoxide dismutase) is preferably from about 20 to about 500 units, more preferably, from 100 to 300, and most preferably about 150 units (i.u.) (McCord/Fridovich units). Preferable sources of SOD for the formulation of the present invention include purified liver SOD, bovine SOD, intercellular SOD, bovine liver SOD, erthrocytic SOD, extracellular SOD, vegetable culture SOD, vegetable SOD, bovine lyophilized liver SOD, fractionated liver SOD, fractionated erythrocyte SOD, and bacterial SOD. The most preferable source of SOD is, however, vegetable SOD, particularly as a food concentrate.

Catalase also stabilizes the formulations of the present invention and compliments SOD. Catalase converts hydrogen peroxide into water and oxygen. It is believed that catalase protects against diseases such as cancer and cardiovascular disorders as well as increasing longevity. The dosage of catalase included is from about 5 mg to 500 units, more preferably, from 100 to 300 units, and most preferably, about 150 units (i.u.) (Baker Units). Preferable sources of catalase include that purified from bovine liver, fractionated liver, bacteria, Aspergillus niger, and vegetables. The most preferable source is, however, vegetables, particularly as a food concentrate.

The core antioxidants can also interact synergistically with the following components, vitamins and minerals which are antioxidants additionally included in the preferred formulation (Core II):
Vitamin A
B-carotene (beta-carotene)
Vitamin E
Ca Ascorbate
Copper
Zinc
Manganese
Selenium
omega-3 fish oil
inositol
PABA
Due to their importance both in the prevention of cancer and cardiovascular disorders, omega-3 fatty acid, PABA and inositol are also an important part of the Core II formulation.

The Core II ingredients may be described as follows:

Vitamin A is a fat soluble vitamin essential in cell differentiation, vision, immunity, healthy skin, hair, and mucous membranes. This vitamin is also necessary for proper reproduction, bone growth and development of teeth. Deficiencies have been associated with a higher incidence of certain cancers. Vitamin A appears to work synergistically with several other vitamins, as well as minerals and antioxidants, to lower cancer rates and to increase longevity. The dose of vitamin A preferably included is from 1,000 to 3,000 i.u., and more preferably, 2,000 i.u. Preferably, the vitamin A source included in the formulation is vitamin A acetate, vitamin A palmitate, retinol, and retinal. The source of vitamin A may be water soluble, fat soluble, or emulsified. Most preferably, however, the vitamin A source is emulsified vitamin A acetate.

Beta carotene or, provitamin A is a precursor to vitamin A. Beta carotene must be converted by the body into active retinol-type vitamin A to be utilized. Beta carotene is also a potent antioxidant and may prevent certain types of cancer. Beta carotene appears to work synergistically with several vitamins, minerals and antioxidants to lower cancer rates and to increase longevity. Although Vitamin A and other retinoids have been shown to inhibit the induction of skin cancer in mice, an unexpected result has been the lack of an inhibitory activity by B-carotene (beta carotene) in the mouse skin carcinogenesis system. However, if B-carotene is given with low doses of vitamin E, BHA or BHT, it very effectively enhances their activity in a synergistic manner. The dosage of beta carotene included is preferably from 2,500 to 7,500 provitamin A units as water soluble beta carotene, or emulsified beta carotene, and most preferably 5,000 provitamin A units as emulsified beta carotene.

Vitamin E is a fat soluble vitamin which functions as an antioxidant protecting lipid membranes against oxidation. Deficiencies in vitamin E can increase the tendency of blood to clot. Vitamin E appears to work synergistically with several vitamins, minerals, e.g., selenium, and antioxidants to lower cancer rates and to increase longevity. The dosage of vitamin E included is preferably from 10 to 100 units as DL-alpha tocopheryl acetate, DL-alpha tocopheryl succinate, water soluble DL-alpha tocopheryl acetate or succinate, emulsified DL-alpha tocopheryl acetate, D-alpha tocopheryl acetate, D-alpha tocopheryl succinate, mixed tocopherols, emulsified D-alpha tocopheryl acetate, emulsified mixed D-tocopherols, water soluble D-alpha tocopheryl acetate, water soluble mixed D-tocopherols, D-alpha tocopherol, emulsified D-alpha tocopherol, water soluble D-alpha tocopherol, or alpha tocopheryl nicotinate. Most preferably, however, the dosage of vitamin E is 100 units as vitamin E, DL-alpha tocopheryl acetate.

Vitamin C is a water soluble essential vitamin. It is critical in producing and maintaining collagen, promotes healing of wounds, aids tooth and bone formation, and is important in producing hormones that regulate basal metabolic rate and body temperature. Vitamin C also acts as an antioxidant and may counteract nitrosamines formed in the intestinal tract, and, therefore, helps reduce the rates of certain kinds of cancers. Vitamin C appears to work synergistically with several other vitamins, as well as minerals and antioxidants, to lower cancer rates and to increase longevity. The dosage of vitamin C included is preferably from 10 to 1,000 mg as ascorbic acid, or salts of ascorbic acid including calcium ascorbate, magnesium ascorbate, sodium ascorbate, potassium ascorbate, esterified ascorbic acid, ascorbyl palmitate. Most preferably, however, the dosage of vitamin C is about 250 mg as calcium ascorbate.

Copper is a trace mineral essential for the formation of red blood cells and the utilization of iron. It also functions in electron transport, connective tissue metabolism and the development of the nervous system. Deficiencies have been linked to nervous system disorders, bone disorders (spontaneous fractures) and anemia. Copper may protect against certain types of cancers. Copper dosage is 0.2 to 20 mg as copper oxide, cupric acetate, cupric butyrate, cupric subcarbonate, cupric chloride, cupric citrate, cupric formate, cupric gluconate, cupric glycinate, cupric hydroxide, cupric oxide, cupric stearate, cupric sulfate, cupric subsulfate, copper gluconate, copper amino acid chelate, copper salicylate, copper proteinate, cupric tyrosinate, copper glycerophosphate, and copper picolinate.

Zinc is a trace mineral essential to cell multiplication, tissue regeneration and wound healing, sexual maturity, and proper growth. Zinc is required for many enzymatic functions throughout the body, and also helps regulate the immune system and insulin metabolism. Preferably, the dosage of zinc included is from about dosage 2 to about 30 mg as zinc oxide, zinc acetate, zinc benzoate, zinc caprylate, zinc carbonate, zinc chloride, zinc citrate, zinc formate, zinc lactate, zinc selenate, zinc stearate, zinc sulfate, zinc picolinate, zinc gluconate, zinc sulfate heptahydrate, zinc sulfate monohydrate, zinc succinate, zinc amino acid chelate, zinc proteinate, zinc fumarate, zinc aspartate, zinc ascorbate, and zinc glycerophosphate. Most preferably, however, the dosage of zinc is 15 mg as zinc oxide.

Manganese is a trace mineral essential to enzyme systems involved in bone development, insulin production as well as other enzyme systems. It is also required for the synthesis of cartilage mucopolysaccharides. Preferably, the manganese dosage is from about 0.5 to about 5 mg as manganese gluconate, manganese acetate, manganese carbonate, manganese chloride, manganese dioxide, manganese hypophosphite, manganese iodide, manganese silicate, manganese sulfate, manganese picolinate, manganese glycerophosphate, manganese lactate, manganese amino acid chelate, manganese proteinate, manganese ascorbate, manganese aspartate, manganese citrate, manganese fumarate, and manganese glycinate. More preferably, the manganese dosage is 2.5 mg as manganese gluconate.

Selenium helps maintain proper immune system function. It is an essential component of glutathione peroxidase, and as an antioxidant helps protect cells from free radical damage and toxic effects of certain chemicals. Deficiencies are linked with increased risk of various cancers, along with increased risk of stroke, angina and heart attack. Selenium appears to work synergistically with several vitamins, minerals and antioxidants to lower cancer rates and to increase longevity. Preferably, the dosage of selenium included is from about 2 µg to about 100 µg as sodium selenate, selenous acid, potassium selenate, selenium oxide, selenomethionine, selenium yeast, selenium vegetable culture, selenium amino acid chelate, selenium proteinate, selenium ascorbate, selenium aspartate, sodium selenite, sodium selenate, potassium selenite, selenocystine, magnesium potassium selenate, magnesium selenate, cupric selenate, cupric selenite, zinc selenate, zinc selenite, calcium selenate, calcium selenite, selenium picolinate, selenium glycinate, and selenium glycerophosphate. Most preferably, however, the dosage of selenium is 25 µg as sodium selenate.

Omega-3 (EPA/DHA) are polyunsaturated fatty acids. Increased dietary levels of polyunsaturated fatty acids reduces platelet aggregation and has been linked to a lower incidence of heart disease and cancer. Omega 3 fish oils, preferably having an EPA:DHA ratio of about 18:12, are included in the supplement of the present invention in a dosage of from 10 to 1,000 mg, more preferably, however, the dosage is from about 50 to about 800 mg, and most preferably 200 mg. The omega-3 fish oil is preferably included as microencapsulated omega-3 fish oils, Omega 3 fish oils, cod liver oil, salmon oil, microencapsulated salmon oil, emulsified cod liver oil, emulsified salmon oil, eicosapentaenoic acid, docosahexenoic acid, emulsified eicosapentaenopic acid, emulsified docosahexaenoic acid, fish oil, EPA, max EPA sardine oil, microencapsulated sardine oil, halibut oil, or microencapsulated halibut oil. Most preferably, however, the omega-3 fish oil is microencapsulated omega-3 fish oil.

Inositol plays a vital metabolic role at the cellular level. Inositol is a bipotropic agent and plays a role in membrane and lipoprotein function. Inositol is preferably included in a dosage of from 5 to 100mg, and most preferably, 50 mg.

According to one embodiment, the daily dietary multi-vitamin and mineral supplement of the present invention further includes para-aminobenzoic acid (PABA) in combination with the preferred amino acids and antioxidants of the present invention. PABA is an essential coenzyme in the metabolism of proteins and in the formation of red blood cells. PABA works synergistically with pantothenic acid. It has been reported that PABA may provide some protection against hypertension and certain cancers. PABA is preferably included in the formulations of the present invention as para-aminobenzoic acid, potassium para-aminobenzoate, sodium para-aminobenzoate, magnesium para-aminobenzoate, calcium para-aminobenzoate. Most preferably, however, PABA is included as the acid in a dosage of from 10 to 100 mg, and most preferably, 40 mg.

Also, the following vitamins and minerals (Core III), although not unique, are necessary and important in this formulation because of their role in normal growth and general bodily needs. Once vitamins have crossed the intestinal walls, many function as co-enzymes essential to all cells. Because they work synergistically, or as a team, it is better to administer them together. A similar synergism is known for many of the minerals. They are as follows:
Folic Acid
Vitamin B₁
Vitamin B₂
Niacinamide
Vitamin B₆
Vitamin B₁₂
Vitamin D₃
Biotin
Ca Pantothenate
Vitamin K₁
Calcium
Iodine
Potassium
Iron
Magnesium
Chromium
Molybdenum
Vanadium
Silicon
Boron

Compositions of the Core III formulations may be described as follows.

Folic Acid is an essential water-soluble vitamin. It is necessary for the synthesis of RNA and DNA, and therefore, vital in cell growth and division. Folic acid is important in red blood cell maturation and in other metabolic processes. The dose of folic acid included is from about 40 to about 1,200 µg as folic acid, and most preferably, 400 µg

Vitamin B₁ (thiamine) is an essential water-soluble vitamin. It is important in the metabolism of carbohydrates and critical for the transmission of high-frequency impulses in the central nervous system as well as maintaining the integrity of the central nervous system. Preferably, the dosage of vitamin B₁ included is from about 1 to about 2 mg as thiamine hydrochloride, thiamine mononitrate, thiamine chloride naphthalene-1, 5-disulfonic acid salt, thiamine triphosphoriate salt, thiamine triphosphate ester, thiamine phosphoric acid ester phosphate salt, thiamine phosphoric acid ester chloride, and thiamine disulfide. Most preferably, however, the dosage of vitamin B₁ is 1.50 mg as thiamine hydrochloride.

Vitamin B₂ (riboflavin) is an essential water-soluble vitamin which is required for the repair and growth of tissues as well as DNA synthesis. Vitamin B₂ assists in metabolizing nutrients, e.g., proteins, fats and carbohydrates, and therefore, is critical in the release of energy. Preferably, the dosage of vitamin B₂ is from about from 170 µg to about 2 mg as riboflavin, riboflavin tetrabutyrate, riboflavin 5' phosphate sodium, and riboflavin monodiethanolamine salt dihydrate. Most preferably, however, the dosage of vitamin B₂ is 1.75 mg as riboflavin.

Niacinamide is critical in the production of energy. Niacinamide is water-soluble, and is an essential constituent of coenzymes I and II that occur in a wide variety of enzyme systems involved in the anaerobic oxidation of carbohydrates. Niacinamide is necessary for DNA formation, and important for the integrity of the central nervous system. Preferably, the dosage of niacinamide included is from about 10 to about 30 mg as niacinamide, and most preferably, 20 mg.

Vitamin B₆ is a term commonly used for a group of three vitamins: pyridoxine, pyridoxal and pyridoxamine. They are important in protein and amino acid metabolism and help to regulate blood glucose levels. These vitamins are needed to synthesize hemoglobin and are important in the proper function of the central nervous system. The dosage of vitamin B₆ is preferably from about 200 µg to about 3 mg as pyridoxine hydrochloride, pyridoxal 5-phosphate, calcium pyridoxine 5-phosphate, pyridoxal. Most preferably, however, the dosage of vitamin B₆ is 20 mg.

Vitamin B₁₂ is an essential water soluble vitamin. It is critical for bone growth, the integrity of the central nerve tissue, normal blood formation, and cell replication and DNA synthesis. Vitamin B₁₂ deficiencies are related to certain anemias. Vitamin B₁₂ is useful especially in the prevention of pernicious anemia. Preferably the dosage of Vitamin B₁₂ included is from about 2 to about 1000 µg as cyanocobalamin, cobalamin, Co-methylcyanocobalamin, zinc-vitamin B₁₂ - tannate, resin bound vitamin B₁₂, and coenzyme B₁₂.

Vitamin D₃ is an oil-soluble vitamin. Vitamin D₃ aids both the absorption of calcium and phosphorus. The primary role of vitamin D₃ is the mineralization of bones and teeth and the regulation of blood calcium levels. Vitamin D₃ is critical for the growth and differentiation of several tissues, and, it is believed, reduces the rate of certain cancers. The dosage of vitamin D₃ included is preferably from about 40 to about 4,000 units as vitamin D₃, water dispersable vitamin D₃, water-soluble vitamin D₃, and emulsified vitamin D₃. Most preferably, however, the dose of vitamin D₃ is 600 units.

Biotin is a water-soluble vitamin essential in metabolism. It is a coenzyme in the synthesis of fatty acids and amino acids. The dosage of biotin is preferably from 10 to 100 µg as biotin, and most preferably, 30 µg.

Calcium pantothenate is a source of pantothenic acid, a water-soluble vitamin. Pantothenic acid is involved with the metabolism of proteins, fats and carbohydrates in the form of exoenzyme A, and is involved in the synthesis of sterols, hormones, porphyrins and acetylcholine. The dosage of calcium pantothenate is preferably from about 1 to about 50mg, and most preferably, 10mg.

Vitamin K₁ is an oil soluble vitamin which is necessary for the formation of prothrombinogen and other blood clotting factors. However, vitamin K-dependent protein in bone suggests a more complex role. Preferably, the dosage of Vitamin K₁ is from about 3 to 100 µg as trans isomer of vitamin K₁, cis-trans isomers of vitamin K₁, emulsified trans isomer of vitamin K₁, emulsified cis-trans isomers of vitamin K₁, water soluble trans vitamin K₁, water soluble cis-trans isomer of vitamin K₁, vitamin K₁ oxide, trans vitamin K₁ oxide, emulsified cis-trans vitamin K₁ oxide, water soluble trans vitamin K₁, water soluble cis-trans vitamin K₁ oxide, vitamin K₂ (2-methyl -3- all trans-polyprenyl-1, 4 napthoquinone) and those compounds of vitamin K₂ which may possess side chains varying in length from C₅ (n=1) to C₆₅ (n=13), water soluble vitamin K₂ and water soluble compounds of K₂ which may possess side chains varying in length from C₅ (n=1) to C₆₅ (n=13), vitamin K₅, dihydrovitamin K₁, diphosphate (0¹, 0⁴ -diphosphoric acid) disodium salt of dihydrovitamin K₁, tetrasodium salt of dihydrovitamin K₁, tetrapotassium salt of dihydrovitamin K₁, diphosphate (0¹, 0⁴ - diphosphoric acid) dipotassium salt of dihydrovitamin K₁, vitamin K₆, vitamin K₇, vitamin K₇ hydrochloride, vitamin K₅ (II), emulsified vitamin K₅ (II), water soluble vitamin K₅ (II). Most preferably, however, the dose of vitamin K₁ is 25 µg.

Calcium is involved with the transmission of nerve impulses, muscle contraction and relaxation, blood clotting, structure and function of cell membranes and B₁₂ absorption. Dietary deficiencies are common and have been associated with accelerated bone loss resulting in alveolar bone loss and accompanying oral health problems, osteoporosis and hypertension. Calcium may also protect against certain type of cancer such as colon cancer. Preferably, the calcium dosage included is from about 40 to about 1,000 mg as dicalcium phosphate dihydrate, dicalcium phosphate, monobasic calcium phosphate, tricalcium phosphate, calcium carbonate, calcium chloride, calcium citrate, calcium fluoride, calcium gluconate, calcium glycerophosphate, calcium hydroxide, calcium lactate, calcium levulinate, calcium sulfate, calcium succinate, calcium ascorbate, calcium fumarate, calcium lysinate, calcium malate, calcium aspartate, calcium amino acid chelate, calcium caseinate, calcium proteinate, calcium picolinate, calcium aspartate, calcium alpha ketoglutarate, calcium caseinate, calcium hydroxide, bone meal, oyster shell, calcium glycerophosphate, calcium hypophosphite, calcium ferrous citrate, calcium formate, calcium hypochlorite, calcium methionate, calcium biphosphate, calcium stearate, calcium tartrate, hydroxyapatite, dolomite, calcium sulfate dihydrate, calcium sulfate hemihydrate, dicalcium phosphate dihydrate, calcium acetate, calcium pyrophosphate, calcium gycinate. More preferable sources of calcium include calcium citrate, calcium carbonate, which act as free radical scavengers, calcium pantothenate, and dicalcium phosphate dihydrate. Dicalcium phosphate dihydrate is a source of calcium and phosphorus which functions in almost all aspects of metabolism, and also aids in the manufacturing (tableting) process.

Iodine is essential for proper thyroid functioning, and therefore, the regulation of the basal metabolic rate. The dosage of iodine included is from about 5 to 500 µg as potassium iodide, sodium iodide, iodinated glycerol, kelp, Atlantic kelp, Pacific kelp, iodine-resublimed, potassium iodate, ferrous iodide, magnesium iodide, manganese iodide, iodine picolinate, iodine aspartate, iodine glycerophosphate. Most preferably, however, the dosage is about 150 µg as potassium iodide.

Potassium is a major intracellular electrolyte. Potassium aids in the transmission of nerve impulses, the release of insulin, and the proper functioning of digestive enzymes. Potassium works with sodium to maintain the balance of body fluids. Preferably, the dosage of 10 to about 1000 mg potassium is from about as potassium chloride, potassium acetate, potassium bicarbonate, potassium carbonate, potassium citrate, potassium gluconate, potassium glycerophosphate, potassium hydroxide, potassium nitrate, dipotassium phosphate, potassium dihydrogen phosphate, tripotassium phosphate, potassium sorbate, potassium sulfate, potassium proteinate, potassium amino acid chelate, potassium fumarate, potassium alpha ketoglutarate, potassium malate, potassium ascorbate, potassium succinate, potassium aspartate, potassium/magnesium aspartate, or potassium picolinate.

Iron is an essential mineral and is needed to transport oxygen to the tissues throughout the body via hemoglobin and myoglobin. Iron is involved in the catalysis of many oxidative reactions within cells and is necessary for the proper functioning of many biochemical reactions. Iron plays a role in final steps of energy metabolism. Iron deficiencies are widespread, especially among women and children. Preferably, the dosage of iron included is from about 2 to about 50 mg as ferrous fumarate, ferrous carbonate mass, ferrous carbonate saccharated, ferrous chloride, ferrous citrate, ferrous gluconate, ferrous iodide, ferrous lactate, ferrous oxide, ferrous succinate, ferrous sulfate, ferrous ascorbate, iron amino acid chelate, ferrous aspartate, ferrous peptonate, iron proteinate, iron chloride, iron reduced, iron oxide saccharated, ferric glycerophosphate, and iron picolinate. Most preferred, however, the dosage of iron is 18 mg as iron gluconate.

Magnesium is a mineral essential for every biological process including glucose metabolism, protein and nucleic acid synthesis, electrical balance of cells, and the transmission of nerve impulses. Magnesium effects the function and structure integrity and contractions of the heart and all other muscles. Dietary deficiencies are widespread. Deficiencies have been linked to cardiovascular diseases. Preferably, the dosage of magnesium included is from about 10 to about 500 mg as magnesium oxide, magnesium benzoate, magnesium carbonate hydroxide, magnesium chloride magnesium citrate, di-basic magnesium citrate, magnesium formate, magnesium hydroxide, magnesium iodide, magnesium lactate, magnesium peroxide, magnesium hydrogenphosphate, trimagnesium phosphate, magnesium silicate, magnesium stearate, magnesium sulfate, magnesium carbonate, magnesium gluconate, magnesium glycerophosphate, magnesium trisilicate, magnesium nicotinate, magnesium malate, magnesium amino acid chelate, magnesium picolinate, magnesium proteinate, magnesium lysinate, magnesium alpha ketoglutarate, and magnesium phosphinate hexahydrate. Most preferably, however, the dosage of magnesium is 100 mg as magnesium oxide.

Chromium is a cofactor for insulin. Chromium is involved in carbohydrate metabolism, especially the utilization of glucose. Low chromium has been linked to diabetes and hypoglycemia. Low chromium has also been associated with heart disease. Chromium deficiencies are widespread. Preferably, the dosage of chromium included is from about 2 to about 50 µg as chromium (+3) chloride, chromic acetate, chromic carbonate, chromic formate, chromic hydroxide, chromic nitrate, chromic oxide, chromic phosphate, chromic potassium sulfate, chromic sulfate, chromic picolinate, chromium polynicotinate, chromium soured yeast, chromium vegetable culture, chromium amino acid chelated, chromium proteinate, chromium aspartate, chromium ascorbate, chromium GTF yeast, chromium glycinate, and chromium glycerophosphate. Most preferably, the dosage of chromium is 25 µg as chromium (+3) chloride.

Molybdenum is considered to be an essential trace mineral. Molybdenum plays a role in iron metabolism. Preferably, the dosage of molybdenum included is from about 2 µg to 100 µg as sodium molybdate, molybdenum amino acid chelate, molybdenum ascorbate, molybdenum yeast, molybdenum vegetable culture, molybdenum disulfide, molybdenum sesquioxide, molybdenum trioxide, sodium phosphomolybdate, molybdenum picolinate, molybdenum glycinate, and molybdenum glycerophosphate. Most preferably, however, the dosage of molybdenum is about 25 µg as sodium molybdate.

Vanadium is a trace mineral found in the diet. Possible biochemical and physiological functions for vanadium have been suggested as including lipid metabolism, glucose metabolism, bone, and tooth metabolism. Vanadium may serve a catalytic function or may be an enzyme cofactor. Preferably, the dosage of vanadium included is from about 2 µg, to about 100 µg as sodium metavanadate, vanadium aspartate, vanadium amino acid chelate, vanadium proteinate, vanadium yeast, vanadium vegetable culture, potassium metavanadate, calcium vanadate, magnesium vanadate, ammonium metavanadate, vanadyl sulfate, oxytartarovanadate, vanadium picolinate, vanadium glycinate, and vanadium glycerophosphate.

Silicon functions as a biologic cross-linking agent that contributes to the structure and resilience of connective tissues, collagen, elastin and mucopolysaccharide. Silicon may be required for bone and cartilage collagen biosynthesis and is apparently involved in bone calcification. Preferably, the dosage of silicon included is from about 2 µg to about 50 µg as sodium metasilicate, silica amino acid chelate, silicon proteinate, silicon plant source, silicon vegetable culture, silicon source trachea, silicon source tendon, silicon source aorta, silicon source mucopolysaccharide, silicon source pectin, silicon source alginic acid, silicic acid, silanolate, silicon dioxide, and silica. More preferably, the dosage of silicon is 10 µg as sodium metasilicate.

Findings suggest that boron influences the metabolism of calcium, phosphorus, magnesium and cholecalciferol. Boron may also be involved in the functional efficiency of membranes. Preferably, the dosage of boron included is from about dosage 500 µg to about 2000 µg as calcium borogluconate, magnesium borogluconate, sodium borate, boric acid, potassium borate, sodium metaborage, boron proteinate, boron amino acid chelate, glyceroborate, magnesium perborate, sodium perborate, calcium perborate, and potassium perborate. Most preferably, however, the dosage of boron is about 1 mg as calcium borogluconate.

With regard to the vitamins and minerals included in the inventive formulation, each (with the possible exception of vitamin A, calcium, magnesium and biotin) is included in an amount equal to or greater than the minimum daily requirement in man. Further, it is preferred that the vitamins and minerals included in the inventive formulation are similar to those included in the multi-vitamin and mineral preparations Centrum® or One-A-Day® (as shown in Table 3).

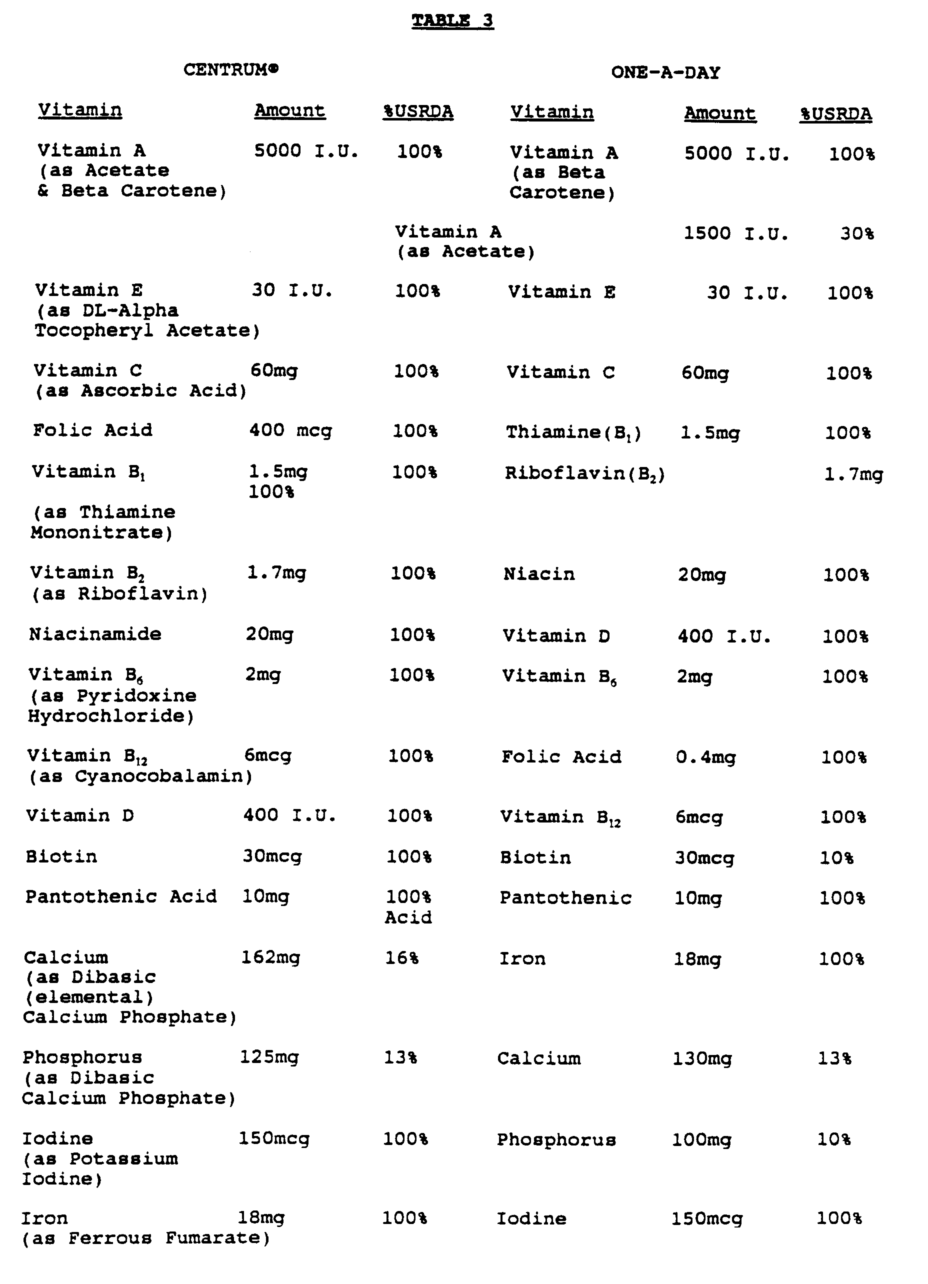

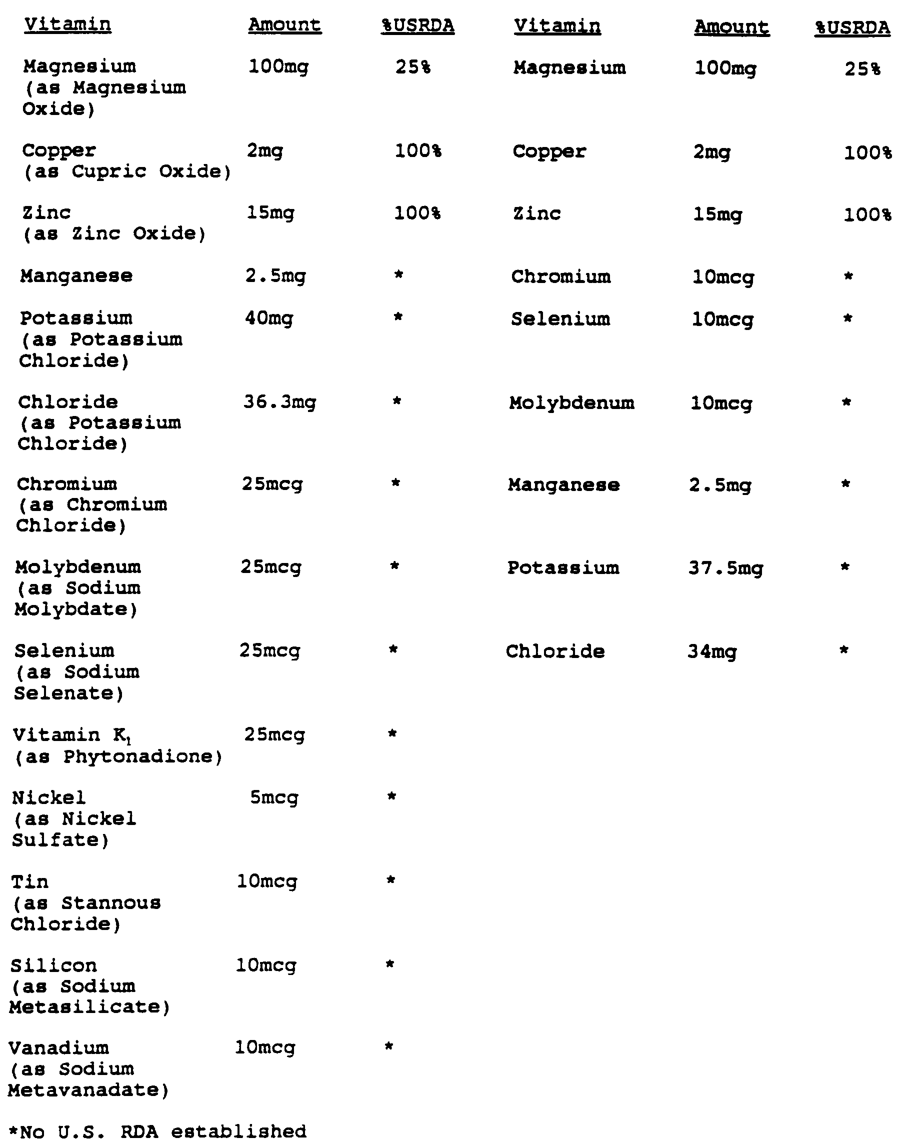

It is known that certain amino acids are essential for proper cellular function. However, it has been suggested by researchers that select amino acids provide some protection against cancer, and may, in fact, act synergistically with the antioxidants of the present invention to protect against cancer. Thus, it is one important aspect of the present invention to provide a daily dietary multi-vitamin and mineral supplement including antioxidants and select amino acids. According to the invention, the antioxidants are selected from the group consisting of BHT, BHA, propyl gallate, and sodium benzoate, and the preferred amino acids or analogs are selected from the group consisting of glutathione, cysteine, methionine, glutamine, and taurine.

Accordingly, one preferred embodiment of the present invention provides a daily dietary multi-vitamin and mineral supplement including, in combination:
(a) from 10 to 300 mg of BHT;
(b) from 10 to 300 mg of BHA;
(c) from 1 to 300 mg of sodium benzoate; and
(d) from 10 to 300 mg of propyl gallate.
(e) from 1 to 20 mg of glutathione;
(f) from 5 to 100 mg of cysteine;
(g) from 1 to 20 mg of methionine;
(h) from 1 to 20 mg of glutamine;
(i) from 2 to 200 mg of bioflavonoids;
(j) from 10 to 500 mg of potassium sorbate/sorbic acid;
(k) from 1 to 20 mg of taurine;
(l) from 100 to 300 units of catalase as a food concentrate; and
(l) from 100 to 300 units of SOD, as a food concentrate.

More preferably, however, the supplement includes:
(a) 50 mg of BHT; (b) 50 mg of BHA;
(c) 4 mg of sodium benzoate; and
(d) 50 mg of propyl gallate.
(e) 10 mg of reduced L-glutathione;
(f) 50 mg of L-cysteine HCl;
(g) 10 mg of DL-methionine;
(h) 10 mg of DL-glutamine; and
(i) 50 mg of bioflavonoids;
(j) 200 mg of potassium sorbate/sorbic acid;
(k) 10 mg of taurine;
(l) 150 units of catalase as a food concentrate; and
(m) 150 units of SOD as a food concentrate.

In addition to the antioxidants and amino acids described above, several other ingredients may be included in the formulations of the invention, including:
(a) omega-3 fish oil;
(b) para-aminobenzoic acid (PABA); and
(c) inositol.

It has been demonstrated that several vitamins and minerals act synergistically in combination with several of the preferred antioxidants and amino acids of the invention; therefore, it is an important aspect of the present invention to provide formulation including these vitamins and minerals in appropriate amounts. Accordingly, a preferred embodiment of the present invention provides a daily dietary multi-vitamin and mineral tablet including, in combination:
(a) from 10 to 300 mg of BHT;
(b) from 10 to 300 mg of BHA;
(c) from 1 to 300 mg of sodium benzoate;
(d) from 10 to 300 mg of propyl gallate.
(e) from 1 to 20 mg of glutathione;
(f) from 5 to 100 mg of cysteine;
(g) from 1 to 20 mg of methionine;
(h) from 1 to 20 mg of glutamine;
(i) from 1 to 20 mg of taurine;
(j) from 1000 to 3000 i.u. of vitamin A;
(k) from 2500 to 7500 i.u. of beta carotene;
(l) from 10 to 100 i.u. of vitamin E;
(m) from 10 to 1000 mg Ca ascorbate; and
(n) from 10 to 500 mg potassium sorbate or sorbic acid.

More preferably, however, the supplement includes:
(a) 50 mg of BHT;
(b) 50 mg of BHA;
(c) 4 mg of sodium benzoate; and
(d) 50 mg of propyl gallate.
(e) 10 mg of reduced L-glutathione;
(f) 50 mg of L-cysteine HCl;
(g) 10 mg of L-methionine;
(h) 10 mg of L-glutamine;
(i) 10 mg of taurine;
(j) 2000 i.u. of vitamin A acetate;
(k) 5000 i.u. of beta carotene;
(l) 100 i.u. of vitamin E, DL alpha tocopheryl acetate;
(m) 250 mg calcium ascorbate; and
(n) 200 mg of potassium sorbate or sorbic acid.

The inventive supplement includes many vitamins and minerals which are commonly used in commercially available multi-vitamin and mineral preparations. Preferably, the supplement of the present invention includes the following vitamins and minerals in an amount equal to or greater than the minimum daily requirement in man:
(a) vitamin C;
(b) folic acid;
(c) vitamin B₁;
(d) vitamin B₂;
(e) niacinamide;
(f) vitamin B₆;
(g) vitamin B₁₂;
(h) vitamin D₃;
(i) biotin;
(j) pantothenic acid;
(k) vitamin K₁;
(l) calcium;
(m) iodine;
(n) potassium;
(o) iron;
(p) magnesium;
(q) copper;
(r) zinc;
(s) manganese;
(t) chromium;
(u) molybdenum;
(v) selenium;
(w) vanadium;
(x) silicon; and
(y) boron.

The formulations of the present invention contain a number of vitamins and minerals similar to other multivitamin formulations, e.g, Centrum®. However, the formulation of the present invention provides those vitamins and minerals in amounts which better reflect the scientific data concerning these agents. For example, the amount of vitamin A included in the present invention is less than in other available formulations because it can be toxic to older people. Further, the amount of beta-carotene included is greater because it is nontoxic and provides improved benefits. For the above reasons the vitamins and minerals included in the present invention offer a broader base of vitamins and minerals than presently available in the industry; thus, providing the public with a comprehensive multivitamin useful in the prevention of heart disease and cancer.

According to one preferred embodiment, the vitamins and minerals included in the inventive formulation includes:

### VITAMINS

(a) Vitamin A acetate from about 1000 to about 3000iu;
(b) Beta carotene from about 3000 to about 6000iu;
(c) Vitamin E, DL alpha tocopheryl acetate from about 20 to about 40 iu;
(d) Calcium ascorbate from about 200 to about 400mg;
(e) Folic acid from about 200 to about 600 µg;
(f) Vitamin B₁ from about 1.0 to about 2mg;
(g) Vitamin B₂ from about 1.5 to about 2mg
(h) Niacinamide from about 10 to about 30mg;
(i) Vitamin B₆ from about 1 to about 3mg;
(j) Vitamin B₁₂ from about 4 to about 8 µg:
(k) Vitamin D₃ from about 400 to about 800iu;
(l) Biotin from about 20 to about 40 µg;
(m) Calcium pantothenate from about 5 to about 15mg; and
(n) Vitamin K₁ from about 15 to about 35 µg.

### MINERALS

(a) dicalcium phosphate from about 50 to about 150 mg;
(b) potassium iodide sufficient to supply from about 100 to about 150 µg of iodine;
(c) potassium chloride sufficient to supply from about 20 to about 60mg of potassium;
(d) ferrous fumarate sufficient to supply from about 16 to about 30mg of iron;
(e) magnesium oxide sufficient to supply from 75 to about 120mg of magnesium;
(f) copper oxide sufficient to supply from about 1 to about 3mg of copper;
(h) zinc oxide sufficient to supply from about 10 to about 20mg of zinc;
(i) manganese gluconate sufficient to supply from about 1 to about 3mg of manganese;
(j) chromium (+3)chloride sufficient to supply from about 15 to about 35 µg of chromium;
(k) sodium molybdate sufficient to supply from about 15 to about 35 µg of molybdenum;
(l) sodium selenate sufficient to supply from about 15 to about 35 µg of selenium;
(m) sodium metavanadate sufficient to supply from about to about 15 µg of vanadium;
(n) sodium metasilicate sufficient to supply from about 5 to about 15 µg of silicon; and
(o) boron from about 1 to about 3mg.

It should be noted, however, that other vitamins and minerals not listed above may be added to the formulations of the present invention without departing from the spirit of the present invention.

However, according to the most preferred embodiment, the vitamins and minerals of the present invention include:

### VITAMINS

(a) 2000 i.u. of vitamin A acetate;
(b) 5000 i.u. of beta carotene;
(c) 100 i.u. of vitamin E, DL alpha tocopheryl acetate;
(d) 250mg of calcium ascorbate;
(e) 400 µg of folic acid;
(f) 1.5mg of vitamin B₁;
(g) 1.75mg of vitamin B₂;
(h) 20mg of niacinamide;
(i) 20mg of vitamin B₆;
(j) 6 µg of vitamin B₁₂;
(k) 600 i.u. of vitamin D₃;
(l) 30mg of biotin;
(m) 10mg of calcium pantothenate; and
(n) 25 µg of vitamin K₁.

### MINERALS

(a) 100mg of dicalcium phosphate;
(b) potassium iodide sufficient to supply 150µg iodine;
(c) potassium chloride sufficient to supply 40mg of potassium;
(d) ferrous gluconate sufficient to supply 18µg of iron;
(e) magnesium oxide sufficient to supply 100mg of magnesium;
(f) copper oxide sufficient to supply 2mg of copper;
(h) zinc oxide sufficient to supply 15mg of zinc;
(i) manganese gluconate sufficient to supply 2.5mg of manganese;
(j) chromium (+3)chloride sufficient to supply 25 µg of chromium;
(k) sodium molybdate sufficient to supply 25 µg of molybdenum;
(l) sodium selenate sufficient to supply 25 µg of selenium;
(m) sodium metavanadate sufficient to supply 10 µg of vanadium;
(n) sodium metasilicate sufficient to supply 10 µg of silicon; and
(o) 1mg of boron.

The protective agents included in the inventive formulations are either already approved by the FDA or generally regarded as safe (GRAS) by the FDA or are generally thought to be safe by scientific experts. These agents include antioxidants, amino acids, and enzymes, which have been shown to be effective in preventing the induction of cancer and cardiovascular disorders as well as extending the life span of experimental animals. Further, a number of the ingredients have been shown to act synergistically with other agents in the formulation. This combined effect should increase their protective effects. For example, selenium and vitamin E work synergistically against the induction of breast cancer. Further, in a number of studies, BHA when given with vitamin A, beta-carotene and vitamin E was shown to provide a protection against cancer. Accordingly, the present invention is directed to a multi-vitamin dietary supplement formulation.

It is believed that the present invention provides a formulation which is cardio- and cancer-protective.

Preferably, the formulation is tableted, and most preferably, the formulation is tableted as a sustained release tablet. The sustained release tablets of the present invention are intended to provide for the extended action of the ingredients from a single dose. Thus, the sustained released tablet is a convenient dosage form by virtue of eliminating the necessity for dosage several times during the day. Moreover, therapeutic benefits may also be obtained by the sustained release of the active ingredients of the inventive formulation. It is believed desireable that the sustained release dosage form of the present invention will provide a slow and constant supply of antioxidants, amino acids, and other beneficial dietary supplements which will provide protection against cancer and cardiovascular disease throughout the day.

In order to stabilize the preparations of the present invention, it has been determined that several of the more reactive constituents are preferably microencapsulated. The microencapsulation of select constituents prevents those constituents from reacting with other ingredients if left unchecked, which would shorten the shelf life of a commercial product embodying the inventive formulation. According to one embodiment, the preferred sulfur-containing amino acids and the polyunsaturated acids are microencapsulated. More specifically, glutathione, cysteine, and omega-3 fish oil are preferably microencapsulated when included in the invented formulation.

According to one preferred embodiment, hydroxypropyl methylcellulose is used as a pharmaceutical adjunct which provides a sustained release of the active constituents of the tablet. According to this preferred embodiment, based upon the physical parameters involved in formation of a tablet which is determined by the nature of the ingredients (compressibility, adhesion of particles, etc.), up to 20% of the dry weight could be required for hydroxypropyl methylcellulose to ensure proper time release tablet. Thus, according to one embodiment, a total formulation weight range, including time released material, would have a weight range of from about 4090 mg to about 3526 mg. Because of the bulk of the formulation, the formulation is preferably produced and administered as 1-3 tablets daily.

The following examples are presented to describe specific preferred embodiments and utilities of the present invention and are not meant to limit the present invention unless otherwise stated in the claims appended hereto.

### EXAMPLES

### In vitro disintegration characteristics

A comparison was made between the relative disintegrations of Centrum tablets and the sustained release tablets of the present invention.

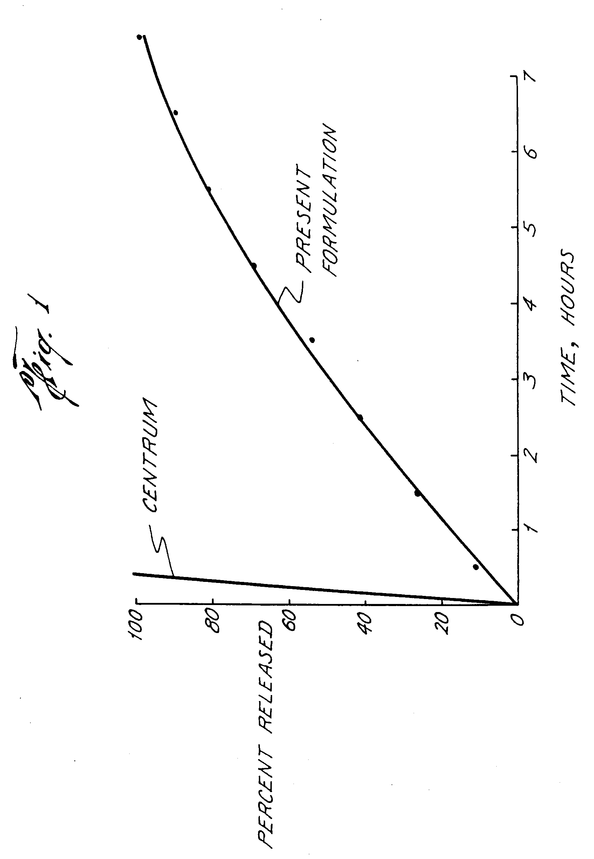

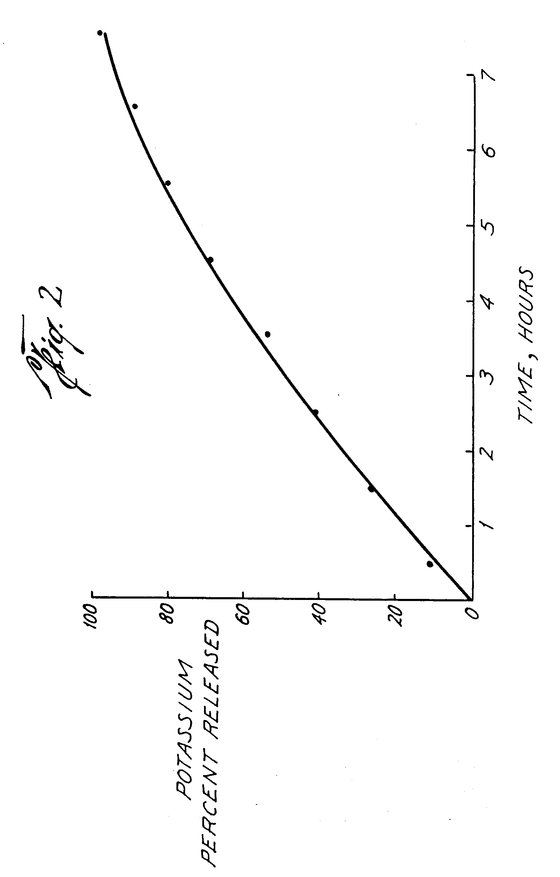

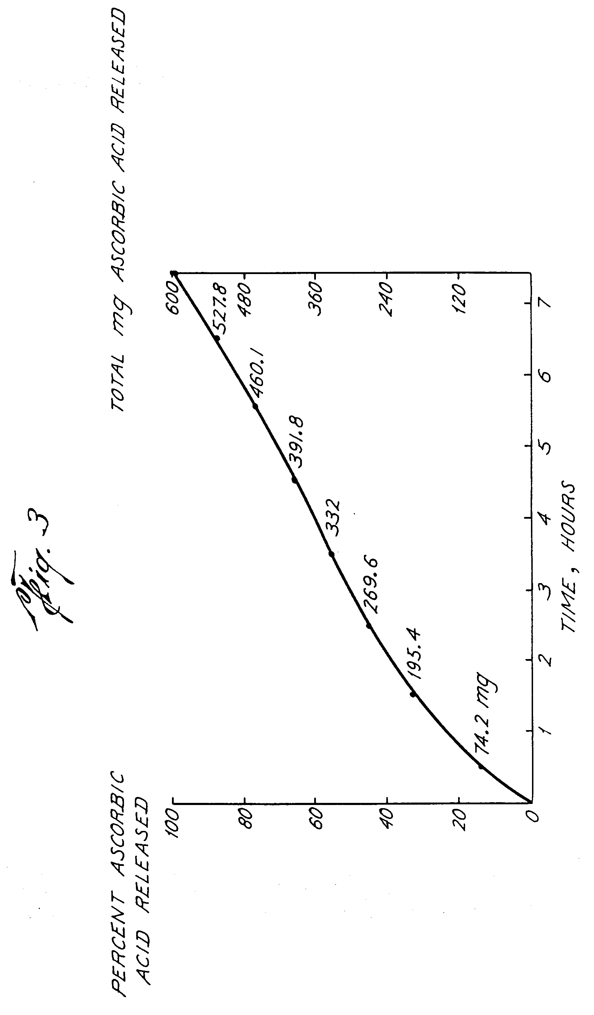

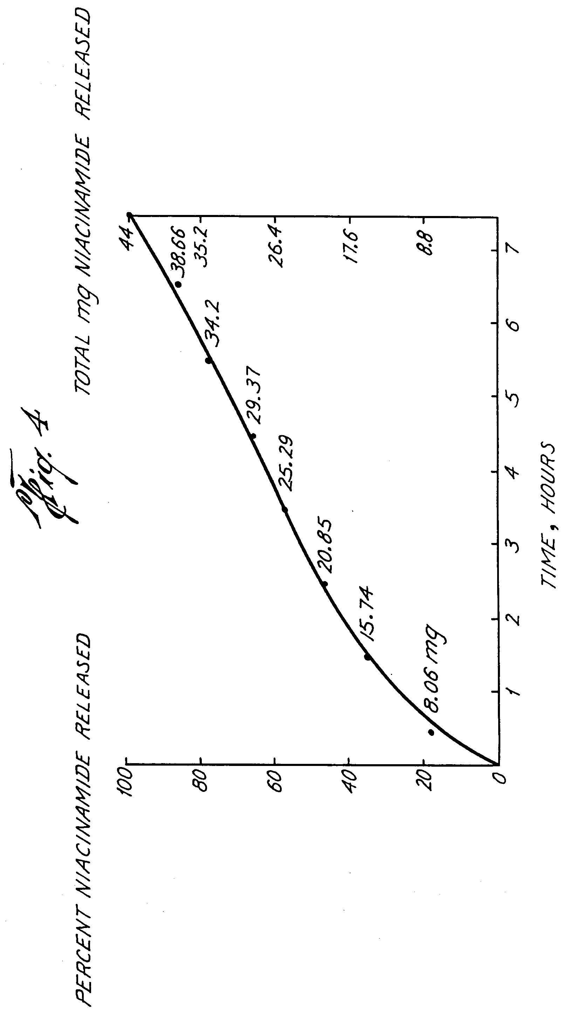

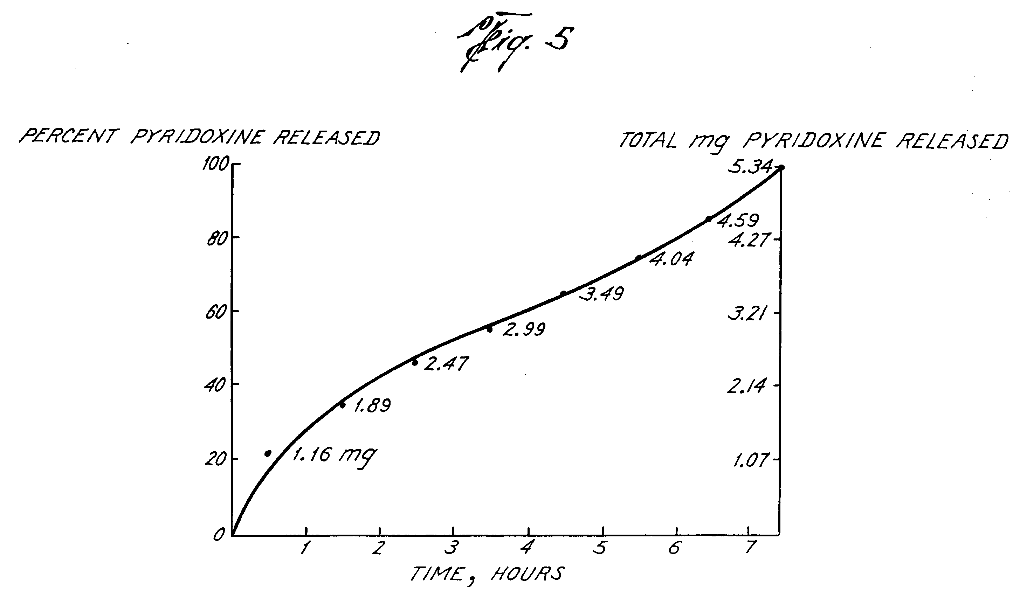

As a measurement of disintegration, the release of four different vitamins, potassium, vitamin C, niacinamide, and pyridoxine was measured. The results, summarized in Figs. 1-6, show that the sustained release tablet of the present invention provides for uniform disintegration over at least seven hours.

As a general procedure, gastric and intestinal juices were prepared according to the United States Pharmacopeia XVIII. The dissolution tests were conducted using the apparatus specified for such procedures in the National Formulary XIII. The apparatus generally consisted of a horizontal rotating shaft to which was attached clamps for holding round cylinders approximately 150mm long and having a diameter of approximately 30 mm wherein the tablets were contained. The apparatus then submerges the tablets in the appropriate dissolution fluid. The temperature of the dissolutions fluid was maintained at 37± 2°C throughout the study. Eight tablets were used to generate the dissolution data. Six Centrum (UDC0005-4239-13 Lederle Laboratories Division American Cyanamid Company) and two sustained release tablets of the present invention, which were prepared as follows.

The following shows the content and instructions for preparing a most preferred formulation (300,000 tablets 3 tablet/dose) of the present invention.

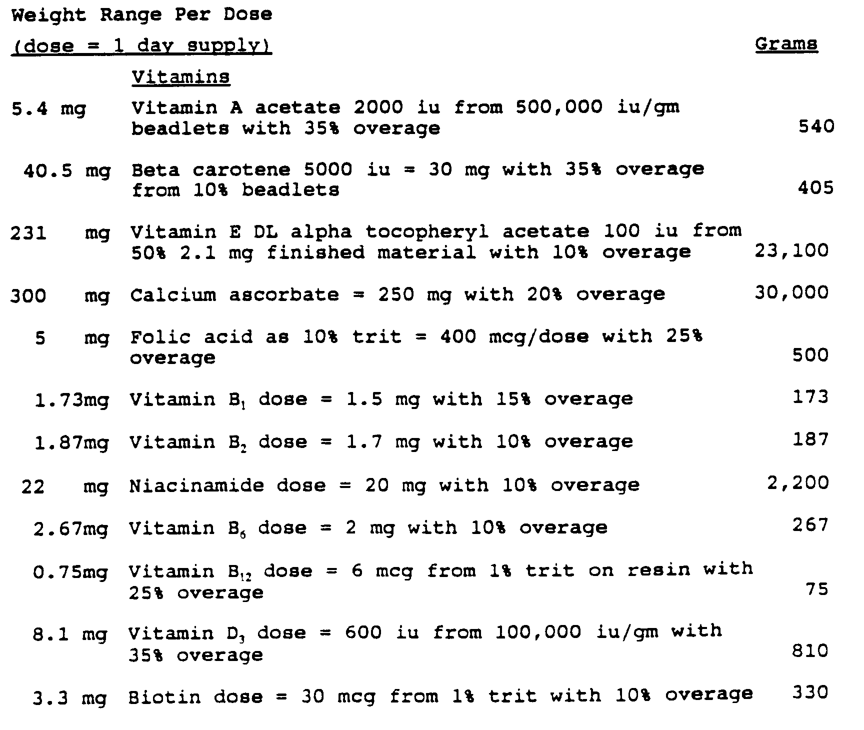

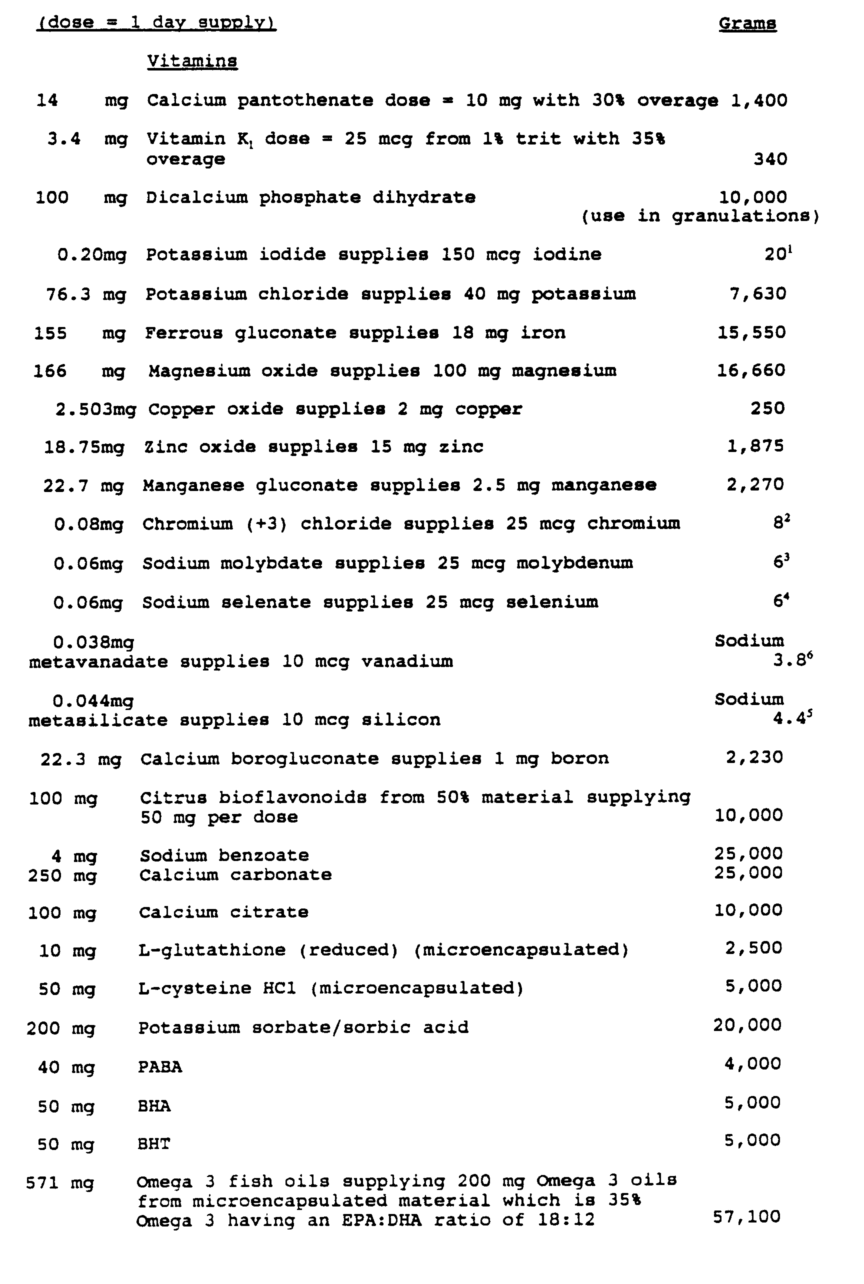

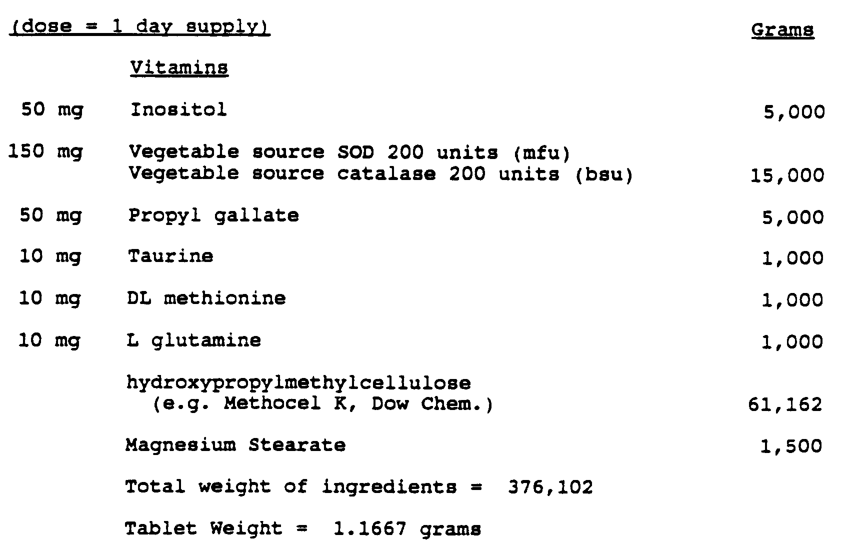

The KI was dissolved in 50 ml H₂O with about 2000 gm of the dicalcium phosphate dihydrate. The mixture was dried at 120°F for 48 hours and then milled. The CrCl₃ was also dissolved in 50 ml H₂O with about 2000 gms dicalcium phosphate dihydrate. This mixture was also dried at 120 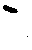 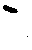 F for about 48 hours and was then milled. The sodium molybdate was dissolved in 50 ml H₂O with about 2000 gm of dicalcium phosphate dihydrate. The mixture was dried at 120 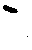 F for about 48 hours and then milled. The sodium selenate was dissolved in 50 ml H₂O with 2000 gms dicalcium phosphate dihydrate and dried at 120°F for about 48 hours and then was milled. The sodium metasilicate was mixed with 2000 gms dicalcium phosphate dihydrate and added to the sodium metavandate which was dissolved in 50 ml H₂O. This mixture was then dried at 120°F for about 48 hours and then was milled. All of the above milled ingredients were then mixed.

All the remaining ingredients were weighed separately and added to a stainless steel ribbon blender and blended for 10 minutes. Tablets were then compressed on a rotary tablet press machine. Tablets weighed 1.2537 grams ± 2%. The tablets were oblong measuring 0.75 inches (height) x 0.35 inches (width) x 0.30 inches (depth). The tablets were produced at 4 tons pressing force per square inch. It was determined that these tablets disintegrated uniformly over an 8 hour period. The tablets are also suitable for an aqueous film coating, such as with riboflavin, in a conventional film coating pan.

The dissolution data was collected at 1/2 hr, 1 and 1/2 hr, 2 and 1/2 hr, 3 and 1/2 hr, 4 and 1/2 hr, 5 and 1/2 hr, 6 and 1/2 hr, and 7 and 1/2 hr. Data for Centrum was obtained for 6 tablets under identical conditions. However, the total dissolution occurred for Centrum at approximately 20 minutes. Therefore, Centrum was only observed in gastric test solution. This data is summarized in Fig. 1.

The release of potassium from the tablet of the present invention was monitored using a Perkin Elmer spectrophotometer. To do this monobase sodium phosphate was used in place of H₂KPO₄ in the intestinal liquid. Replacement of H₂KPO₄ with H₂NaPO₄ allowed accurate measurement of potassium from the tablet without interferences.

Measurements of potassium were made at 766.5 lambda using atomic absorption. Results:

### Time %K released during observed interval

| | |
|---|---|
| 0 - 1/2hr | 11.54 |
| 1/2 - 2 1/2hr | 14.61 |
| 1/2 - 2 1/2hr | 14.61 |
| 2 1/2 - 3 1/2hr | 14.61 |
| 3 1/2 - 4 1/2hr | 13.15 |
| 4 1/2 - 5 1/2hr | 13.15 |
| 5 1/2 - 6 1/2hr | 10.96 |
| 6 1/2 - 7 1/2hr | 7.30 |

To obtain interval values intestinal fluid was replaced hourly. The above data is summarized in Fig. 2.

Data for ascorbic acid, niacinamide, and pyridoxine HCl were generated using a Bausch and Lomb spectrophotometer (Model 21). The wavelengths used for analysis were:
- 245mm: Ascorbic Acid
- 261mm: Niacinamide
- 291mm: Pyridoxine
Samples were taken from 1/2hr, 1/2hr, 2 1/2hr, 3 1/2hr, 4 1/2hr, 5 1/2hr, 6 1/2hr, 7 1/2hr periods. This data is summarized in Figs. 3, 4, and 5 respectively. The data collected showed that the tablet of the present invention provided for the sustained release of the constituents of the inventive formulation.

## Claims

1. A daily dietary multi-vitamin and mineral supplement for a human comprising, in combination:
at least one antioxidant selected from the group consisting of BHT, BHA, propyl gallate, and sodium benzoate;
vitamin A;
vitamin E; and
beta carotene.

2. The supplement of claim 1 comprising BHT, BHA, propyl gallate, and sodium benzoate.

3. The supplement of claim 2 further defined as comprising: Vitamin C, Selenium, Copper, Zinc, Manganese, Omega-3 fish oil, Inositol, PABA, Bioflavonoids, Reduced L-glutathione, L-cysteine, Potassium sorbate or sorbic acid, Sodium benzoate, Taurine, D L-Methionine, L-glutamine, SOD, and catalase.

4. The supplement of claim 3 further defined as comprising: Folic Acid, Vitamin B₁, Vitamin B₂, Niacinamide, Vitamin B₆, Vitamin B₁₂, Vitamin D₃, Biotin, Ca Pantothenate, Vitamin K₁, Calcium, Iodine, Potassium, Iron, Magnesium, Chronium, Molybdenum, Vanadium, Silicon and Boron.

5. The supplement of any previous claim comprising:
at least one amount of an antioxidant selected from the group consisting of 10 to 300 mg BHT, 10 to 300 mg BHA, 10 to 300 mg propyl gallate, and 1 to 300 mg sodium benzoate;
1000 to 3000 i.u. vitamin A;
10 to 100 i.u. vitamin E; and
2500 to 7500 i.u. beta carotene.

6. The supplement of any previous claim comprising 10 to 300 mg BHT, 10 to 300 mg BHA, 10 to 300 mg propyl gallate, and 1 to 300 mg sodium benzoate.

7. The supplement of claim 6 comprising:
from 10 to 1000 mg of vitamin C;
from 2 µg to 100 µg of sodium selenate;
from 1 to 20 mg of reduced 1-glutathione;
from 5 to 100 mg of L-cysteine;
from 1 to 20 mg of D,L-methionine;
from 1 to 20 mg of L-glutamine;
from 1 to 20 mg of taurine;
from 10 to 100 mg of PABA;
from 10 to 1000 mg of Omega-3 fish oil having an EPA:DHA ratio of about 18:12;
from 100 to 300 i.u. of superoxide dismutase;
from 2 to 200 mg of a bioflavonoid;
from 100 to 300 i.u. of catalase;
from 5 to 100 mg of inositol; and
from 10 to 500 mg of potassium sorbate or sorbic acid.

8. The supplement of any previous claim comprising:
at least one amount of an antioxidant selected from the group consisting of 50 mg BHA, 50 mg BHT, 50 mg propyl gallate, and 4 mg sodium benzoate;
2000 i.u. vitamin A;
100 i.u. vitamin E; and
5000 i.u. beta carotene.

9. The supplement of any previous claim comprising 50 mg BHA, 50 mg BHT, 50 mg propyl gallate, and 4 mg sodium benzoate.

10. The supplement of claim 9 comprising:
250 mg of vitamin C;
sodium selenate sufficient to supply about 25 µg of selenium;
10 mg of reduced L-glutathione;
50 mg of L-cysteine HCl;
10 mg of L-methionine;
10 mg of L-glutamine;
10 mg of taurine;
40 mg of PABA;
200 mg of Omega-3 fish oil having an EPA:DHA ratio of about 18:12;
150 i.u. of superoxide dismutase;
50 mg of a bioflavonid;
150 i.u. of catalase;
50 mg of inositol; and
200 mg of potassium sorbat or sorbic acid.

11. The supplement of any of the preceding claims comprising:
2,000 I.U. Vitamin A acetate;
5,000 I.U. Beta Carotene (provitamin A units);
100 I.U. Vitamin E (DL-alpha tocopheryl acetate);
250 mg Calcium ascorbate;
400 µg Folic Acid;
1.5 mg Vitamin B1;
1.75 mg Vitamin B2;
20 mg Niacinamide;
20 mg Vitamin B6;
6 µg Vitamin B12;
600 I.U. Vitamin D3;
30 mg Biotin;
10 mg Calcium pantothenate;
150 µg Iodine (potassium iodide);
18 mg Iron (ferrous gluconate);
100 mg Dicalcium phosphate;
100 mg Magnesium (oxide);
2 mg Copper (oxide);
15 mg Zinc (oxide);
25 µg Vitamin K1;
40 mg Potassium (Potassium chloride);
25 µg Chromium (chloride);
2.5 mg Manganese (gluconate);
25 µg Molybdenum (sodium molybdate);
25 µg Selenium (sodium selenate);
200 mg omega 3 Oils (EPA:DHA ratio of 18:12);
10 µg Vanadium (sodium metavanadate);
1 mg Boron (calcium borogluconate);
10 µg Silicon (sodium metasilicate);
50 mg Citrus Bioflavonoids;
4 mg Sodium Benzoate;
200 mg Potassium Sorbate;
50 mg BHT (butylated hydroxytoluene);
50 mg Inositol;
50 mg Propyl gallate;
50 mg L-Cystein HCl;
10 mg L-Glutathione;
10 mg L-Methionine;
10 mg L-Glutamine; and
10 mg Taurine.

12. An oral sustained release tablet including the dietary multi-vitamin and mineral supplement according to any previous claim.

## Patentansprüche

1. Täglicher, dietätischer Multivitamine und Mineralien enthaltender Nahrungszusatz für einen Menschen, in Kombination enthaltend:
mindestens ein Antioxidanz aus der Gruppe, bestehend aus BHT, BHA, Propylgallat und Natriumbenzoat;
Vitamin A;
Vitamin E;
und Beta-Carotin.

2. Der Nahrungszusatz gemäß Anspruch 1, wobei er BHT, BHA Propylgallat und Natriumbenzoat enthält.

3. Der Nahrungszusatz gemäß Anspruch 2, wobei er ferner folgende Stoffe enthält: Vitamin C, Selen, Kupfer, Zink, Mangan, Omega-3-Fischöl, Inosit, PABA, Bioflavonoide, reduziertes L-Glutathion, L-Cystein, Kaliumsorbat oder Sorbinsäure, Natriumbenzoat, Taurin, D,L-Methionin, L-Glutamin, SOD und Catalase.

4. Der Nahrungszusatz gemäß Anspruch 3, der außerdem folgende Stoffe enthält: Folsäure, Vitamin B₁, Vitamin B₂, Niacinamid, Vitamin B₆, Vitamin B₁₂, Vitamin D₃, Biotin, Calzium Pantothenat, Vitamin K₁, Calzium, Jod, Kalium, Eisen, Magnesium, Chrom, Molybdän, Vanadium, Silicon und Bor.

5. Der Nahrungszusatz gemäß einem oder mehreren der vorstehenden Ansprüche, wobei er folgendes enthält:
mindestens eine Menge eines Antioxidants aus der Gruppe, bestehend aus 10 bis 300 mg BHT, 10 bis 300 mg BHA, 10 bis 300 mg Propylgallat und 1 bis 300 mg Natriumbenzoat;
1000 bis 3000 i. u. Vitamin A;
10 bis 100 i. u. Vitamin E;
und 2500 bis 7500 i. u. Beta Carotin.

6. Der Nahrungszusatz gemäß einem oder mehreren der vorstehenden Ansprüche, der 10 bis 300 mg BHT, 10 bis 300 mg BHA, 10 bis 300 mg Propylgallat und 1 bis 300 mg Natriumbenzoat enthält.

7. Der Nahrungszusatz gemäß Anspruch 6 wobei er folgende Stoffe enthält:
10 bis 1000 mg Vitamin C;
2 µg bis 100 µg Natriumselenat;
1 bis 20 mg reduziertes L-Glutathion;
5 bis 100 mg L-Cystein;
1 bis 20 mg D,L-Methionin;
1 bis 20 mg L-Glutamin;
1 bis 20 mg Taurin;
10 bis 100 mg PABA;
10 bis 1000 mg Omega-3-Fischöl mit einem EPA:DHA-Verhältnis von ∼ 18:12;
100 bis 300 i. u. Superoxiddismutase;
2 bis 200 mg Bioflavonoid;
100 bis 300 i. u. Katalase;
5 bis 100 mg Inosit;
und 10 bis 500 mg Kaliumsorbat oder Sorbinsäure.

8. Der Nahrungszusatz gemäß einem oder mehreren der vorstehenden Ansprüche, wobei er folgendes enthält:
mindestens eine Menge eines Antioxidants aus der Gruppe, bestehend aus 50 mg BHA, 50 mg BHT, 50 mg Propylgallat und 4 mg Natriumbenzoat;
2000 i. u. Vitamin A;
100 i. u. Vitamin E;
und 5000 i. u. Beta-Carotin.

9. Der Nahrungszusatz gemäß einem oder mehreren der vorstehenden Ansprüche, wobei er 50 mg BHA, 50 mg BHT, 50 mg Propylgallat und 4 mg Natriumbenzoat enthält.

10. Der Nahrungszusatz gemäß Anspruch 9, wobei er folgende Stoffe enthält:
250 mg Vitamin C;
genügend Natriumselenat um ∼ 25 µg Selen zur Verfügung zu stellen;
10 mg reduziertes L-Glutathion;
50 mg L-Cystein HCL;
10 mg L-Methionin;
10 mg L-Glutamin;
10 mg Taurin;
40 mg PABA;
200 mg Omega-3-Fischöl mit einem EPA:DHA-Verhältnis von ∼ 18:12;
150 i. u. Superoxiddismutase;
50 mg Bioflavonoid;
150 i. u. Katalase;
50 mg Inosit;
und 200 mg Kaliumsorbat oder Sorbinsäure.

11. Der Nahrungszusatz gemäß einem oder mehreren der vorstehenden Anprüche, der aus folgenden Stoffen besteht:
2000 I. U. Vitamin A-Acetat;
5000 I. U. Beta-Carotin (Provitamin A-Einheiten);
100 I. U. Vitamin E (DL-Alpha Tocopheryl Acetat);
250 mg Calciumascorbat;
400 µg Folsäure;
1,5 mg Vitamin B₁;
1,75 mg Vitamin B₂;
20 mg Niacinamid;
20 mg Vitamin B₆;
6 µg Vitamin B₁₂;
600 I. U. Vitamin D₃;
30 mg Biotin;
10 mg Calciumpantothenat;
150 µg Jod (Kaliumjodid);
18 mg Eisen (Eisengluconat);
100 mg Dicalciumphosphat;
100 mg Magnesium (Oxid);
2 mg Kupfer (Oxid);
15 mg Zink (Oxid);
25 µg Vitamin K₁;
40 mg Kalium (Kaliumchlorid);
25 µg Chrom (Chlorid);
2,5 mg Mangan (Gluconat);
25 µg Molybdän (Natriummolybdat);
25 µg Selen (Natriumselenat);
200 mg Omega-3-Öle (EPA:DHA im Verhältnis von 18:12);
10 µg Vanadium (Natriummetavanadat);
1 mg Bor (Calciumborogluconat);
10 µg Silicium (Natriummetasilicat);
50 mg Citrusbioflavonoide;
4 mg Natriumbenzoate;
200 mg Kaliumsorbat;
50 mg BHT (butyliertes Hydroxytoluol);
50 mg Inosit;
50 mg Propylgallat;
50 mg L-Cystein HCL;
10 mg L-Glutathion;
10 mg L-Methionin;
10 mg L-Glutamin;
und 10 mg Taurin.

12. Eine orale Tablette mit verzögerter Freisetzung, die den diätetischen Mulitivitamin- und Mineralien Nahrungszusatz gemäß einem oder mehreren der vorstehenden Ansprüche enthält.

## Revendications

1. Complément alimentaire, multi-vitaminé et minéralisé, journalier pour un sujet humain, comprenant, en combinaison :
au moins un agent antioxydant choisi parmi le groupe constitué du BHT, du BHA, du gallate de propyle et du benzoate de sodium;
de la vitamine A;
de la vitamine E; et
du bêta-carotène.

2. Complément selon la revendication 1 comprenant du BHT, du BHA, du gallate de propyle et du benzoate de sodium.

3. Complément selon la revendication 2, défini davantage comme comprenant : de la vitamine C, du sélénium, du cuivre, du zinc, du manganèse, de l'huile de poisson de type oméga-3, de l'inositol, du PABA, des bioflavonoïdes, du L-glutathion réduit, de la L-cystéine, du sorbate de potassium ou de l'acide sorbique, du benzoate de sodium, de la taurine, de la DL-méthionine, de la L-glutamine, de la SOD et de la catalase.

4. Complément selon la revendication 3, défini davantage comme comprenant : de l'acide folique, de la vitamine B₁, de la vitamine B₂, de la nicotinamide, de la vitamine B₆, de la vitamine B₁₂, de la vitamine D₃, de la biotine, du pantothénate de Ca, de la vitamine K₁, du calcium, de l'iode, du potassium, du fer, du magnésium, du chrome, du molybdène, du vanadium, du silicium et du bore.

5. Complément selon l'une quelconque des revendications précédentes, comprenant :
au moins une quantité d'un agent antioxydant choisi parmi le groupe constitué de 10 à 300 mg de BHT, de 10 à 300 mg de BHA, de 10 à 300 mg de gallate de propyle et de 1 à 300 mg de benzoate de sodium;
1000 à 3000 IU de vitamine A;
10 à 100 IU de vitamine E; et 2500 à 7500 IU de bêta-carotène.

6. Complément selon l'une quelconque des revendications précédentes comprenant 10 à 300 mg de BHT, 10 à 300 mg de BHA, 10 à 300 mg de gallate de propyle et 1 à 300 mg de benzoate de sodium.

7. Complément selon la revendication 6, comprenant :
de 10 à 1000 mg de vitamine C;
de 2 µg à 100 µg de séléniate de sodium;
de 1 à 20 mg de L-glutathion réduit;
de 5 à 100 mg de L-cystéine;
de 1 à 20 mg de DL-méthionine;
de 1 à 20 mg de L-glutamine;
de 1 à 20 mg de taurine;
de 10 à 100 mg de PABA;
de 10 à 1000 mg d'huile de poisson de type oméga-3, présentant un rapport EPA : DHA d'environ 18 : 12;
de 100 à 300 IU de superoxyde dismutase;
de 2 à 200 mg de bioflavonoïde;
de 100 à 300 IU de catalase;
de 5 à 100 mg d'inositol; et
de 10 à 500 mg de sorbate de potassium ou d'acide sorbique.

8. Complément selon l'une quelconque des revendications précédentes, comprenant :
au moins une quantité d'un agent antioxydant choisi parmi le groupe constitué de 50 mg de BHA, de 50 mg de BHT, de 50 mg de gallate de propyle et de 4 mg de benzoate de sodium;
2000 IU de vitamine A;
100 IU de vitamine E; et
5000 IU de bêta-carotène.

9. Complément selon l'une quelconque des revendications précédentes comprenant 50 mg de BHA, 50 mg de BHT, 50 mg de gallate de propyle et 4 mg de benzoate de sodium.

10. Complément selon la revendication 9, comprenant :
250 mg de vitamine C;
suffisamment de séléniate de sodium pour fournir environ 25 µg de sélénium;
10 mg de L-glutathion réduit;
50 mg de L-cystéine HCl;
10 mg de L-méthionine;
10 mg de L-glutamine;
10 mg de taurine;
40 mg de PABA;
200 mg d'huile de poisson de type oméga-3, présentant un rapport EPA : DHA d'environ 18 : 12;
150 IU de superoxyde dismutase;
50 mg de bioflavonoïde;
150 IU de catalase;
50 mg d'inositol; et
200 mg de sorbate de potassium ou d'acide sorbique.

11. Complément selon l'une quelconque des revendications précédentes, comprenant :
2000 IU d'acétate de vitamine A;
5000 IU de bêta-carotène (unités de provitamine A);
100 IU de vitamine E (acétate de DL-alpha-tocophéryle);
250 mg d'ascorbate de calcium;
400 µg d'acide folique;
1,5 mg de vitamine B₁;
1,75 mg de vitamine B₂;
20 mg de nicotinamide;
20 mg de vitamine B₆;
6 µg de vitamine B₁₂;
600 IU de vitamine D₃;
30 mg de biotine;
10 mg de pantothénate de calcium;
150 µg d'iode (iodure de potassium);
18 mg de fer (gluconate ferreux);
100 mg de phosphate dicalcique;
100 mg (d'oxyde) de magnésium;
2 mg (d'oxyde) de cuivre;
15 mg (d'oxyde) de zinc;
25 µg de vitamine K₁;
40 mg de potassium (chlorure de potassium);
25 µg (de chlorure) de chrome;
2,5 mg (de gluconate) de manganèse;
25 µg de molybdène (molybdate de sodium);
25 µg de sélénium (séléniate de sodium);
200 mg d'huiles de type oméga-3 (rapport EPA : DHA de 18 : 12);
10 µg de vanadium (métavanadate de sodium);
1 mg de bore (borogluconate de calcium);
10 µg de silicium (métasilicate de sodium);
50 mg de bioflavonoïdes d'agrumes;
4 mg de benzoate de sodium;
200 mg de sorbate de potassium;
50 mg de BHT (hydroxytoluène butylé);
50 mg d'inositol;
50 mg de gallate de propyle;
50 mg de L-cystéine HCl;
10 mg de L-glutathion;
10 mg de L-méthionine;
10 mg de L-glutamine; et
10 mg de taurine.

12. Comprimé oral à libération prolongée, comprenant le complément alimentaire multi-vitaminé et minéralisé selon l'une quelconque des revendications précédentes.
